Europäisches Patentamt

European Patent Office

Office européen des brevets

(11) Publication number: **0 284 249**
**A1**

## EUROPEAN PATENT APPLICATION

(21) Application number: 88302104.0

(22) Date of filing: 10.03.88

(51) Int. Cl.⁴ **A61K 9/14** , A61K 47/00 , A61K 45/02 , A61K 37/02

(30) Priority: 13.03.87 US 25310

(43) Date of publication of application:
**28.09.88 Bulletin 88/39**

(84) Designated Contracting States:
**AT BE CH DE ES FR GB GR IT LI LU NL SE**

(71) Applicant: **INTERFERON SCIENCES, INC.**
**783 Jersey Avenue**
**New Brunswick New Jersey 08901(US)**

(72) Inventor: **Bontempo, John A.**
**9 Candle Lane**
**East Brunswick New Jersey 08816(US)**
Inventor: **Evans, Sean A.**
**1005 Normandy Court**
**Flemington New Jersey 08822(US)**

(74) Representative: **Bannerman, David Gardner et al**
**Withers & Rogers 4 Dyer's Buildings Holborn**
**London, EC1N 2JT(GB)**

(54) Lyophilized lymphokine composition.

㊾ An exceptionally stable lyophilized lymphokine composition for therapeutic administration upon reconstitution is disclosed which comprises:

a) a therapeutically effective amount of a lymphokine, for example, an interferon such as recombinant alpha-2 interferon;

b) a bulking agent such as mannitol or human serum albumin;

c) a stabilizer for the lymphokine such as glycine or human serum albumin;

d) a dispersant such as polysorbate 20;

e) an isotonic agent such as sodium chloride; and,

f) a buffer such as a combination of succinic acid and sodium succinate to maintain the pH of the composition within a range which is conducive to the stability of the lymphokine component thereof.

EP 0 284 249 A1

# LYOPHILIZED LYMPHOKINE COMPOSITION

## BACKGROUND OF THE INVENTION

This invention relates to therapeutic compositions based on lymphokines such as the interleukins and the interferons, especially alpha-interferons of both the naturally occurring and recombinant types, and in particular, lyophilized preparations of such compositions possessing excellent storage stability at ambient temperatures over prolonged periods.

The lymphokines generally (of which the interferons are at present the most notable representatives) are water-soluble proteins derived from human cells or gene-recombining microbial cells and have been widely investigated for their therapeutic effects on various human diseases. However, the lymphokines are relatively unstable and special precautions must be observed in their isolation, formulation and storage if predictable levels of potency are to be reliably achieved.

Lyophilization of lymphokines such as the interferons and the interleukins to render them stable and thus preserve their potency prior to administration has been practiced.

U.S. Patent No. 4,469,228 describes a kit for preparing a lyophilized alpha interferon gel formulation suitable for topical administration. The preferred lyophilized interferon is said to be disclosed in United States patent application Serial No. 466,707 of February 15, 1983 which lies in a chain of applications underlying the grant of U.S. Patent No. 4,496,537. The latter patent describes a lyophilized alpha-interferon formulation containing glycine or alanine for improved biological stability and, optionally, human serum albumin and appears to cover INTRON A (Schering Corporation, Kenilworth, NJ), an injectable lyophilized recombinant alpha-2b interferon. The package insert for INTRON A calls for storing the preparation at 2-8°C (36-46°F).

U.S. Patent No. 4,604,377 describes a lyophilized formulation of selectively oxidized microbially produced recombinant interleukin-2(IL-2) in which the recombinant IL-2 is admixed with a water soluble carrier such as mannitol that provides bulk and a sufficient amount of sodium dodecyl sulfate to ensure the solubility of the recombinant IL-2 in water.

European Patent application 0 080 879 describes an interferon preparation stabilized with a polyhydric sugar alcohol such as mannitol and also mentions the addition of buffers and anionic surfactants. No mention is made of a lyophilized interferon preparation.

European Patent Application 0 123 291 discloses a lyophilized interferon preparation stabilized with an alkali metal salt such as sodium chloride, or a saccharide such as dextran, chondroitin sulfuric acid, starch, glycogen, inulin, alginic acid salts, etc., and optionally, human serum albumin (HSA).

Laid Open Japanese Patent Publication No. 1980/102,519 describes a lyophilized interferon preparation containing, inter alia, polyoxyethylene-polyoxypropylene copolymers, polyethylene glycol and chelating agents such as NaEDTA.

The product insert for Roferon-A (Roche Laboratories Division of Hoffmann-LaRoche Nutley, NJ) describes an injectable interferon preparation, believed to be lyophilized and containing phenol as a preservative, which like INTRON A, should be stored at 2-8°C (36-46°F).

## SUMMARY OF THE INVENTION

In accordance with the present invention, a lyophilized lymphokine composition for therapeutic administration exhibits enhanced storage stability at ambient temperature is provided which comprises:

a) a therapeutically effective amount of a lymphokine;

b) a bulking agent;

c) a stabilizer for the lymphokine;

d) a dispersant;

e) an isotonic agent; and,

f) a buffer to maintain the pH of the composition within a range which is conducive to the stability of the lymphokine component thereof.

Unlike the lyophilized interferon preparations of INTRON A and Roferon-A referred to above whose package inserts recommend storage at 2-8°C, the lyophilized lymphokine composition of the present invention substantially retains its initial biological activity for periods of at least six months, and commonly up to two years or more, when stored at ambient temperatures (e.g., at about 80°F).

Therapeutic formulations which do not require refrigeration are generally preferred over those that do (other things being equal) for reasons which are readily apparent. Aside from the added expense of refrigeration, there is always the problem that an interruption in refrigeration resulting from a mechanical malfunction, power outage or any other cause will result in spoilage of the therapeutic composition or a dimunition in its potency, the extent of the latter usually being unascertainable merely on inspection. There is also the problem of the relative unavailability of refrigeration in various regions of the world, especially in the tropics.

Moreover, where it is desired to provide the lyophilized therapeutic preparation as part of a single sealed package, or kit, containing the means for its reconstitution and administration (e.g., a two-component mixing syringe in the case of a parenterally administered preparation), any requirement that the therapeutic be refrigerated (the preferred storage condition for the interferon preparation disclosed in U.S. Patent No.4,469,228 discussed supra) can be particularly disadvantageous given the relative bulkiness of such a package which is bound to be much larger than the container holding the therapeutic. Storage of any reasonable quantity of the packages would necessarily take up a disproportionate amount of available refrigerator volume. Such an inefficient use of refrigeration is a factor which tends to militate against providing lyophilized therapeutics which require refrigeration in the form of a unitary, sealed administrative package. By contrast, storage of such a package at ambient temperature, assuming, of course, the therapeutic component thereof can be properly maintained this way, would not be nearly as inconvenient.

Accordingly, the lyophilized lymphokine composition of this invention and its packaging in the form of a kit containing everything necessary for its reconstitution and parenteral administration is believed to represent a significant advance over the lyophilized interferon preparations of the prior art referred to above.

## DESCRIPTION OF THE PREFERRED EMBODIMENTS

While the invention herein is applicable to all water-soluble lymphokines, it will be particularly illustrated in connection with the interferons, cell-elaborated substances which possess a glycoprotein structure. Examples of interferons which are suitable for lyophilization in accordance with the present invention include natural interferon, interferon produced by incubation of animal cells, interferon produced by incubation of a mircoorganism obtained by recombinant DNA technology, or by known techniques of cell fusion, etc., and also interferon of any of types alpha, beta or gamma. Combinations of lymphokines, for example, two or more different kinds of interferon, mixtures of one or more interferons with one or more lymphokines of another class such as tumor necrosis factor (TNF) are also contemplated. Alpha-2 interferon obtained, for example, by the recombinant-DNA methods disclosed by Nagata et al., Nature Vol. 284, pp. 316-320 (1980), has been selected as the specific interferon for illustrating the present invention.

The amount of interferon to be formulated in the lyophilized composition herein is not critical but will be selected based on the particular therapeutic effect desired. In general, the lyophilized interferon composition can be formulated in such a manner that upon reconstitution, it will possess an activity of from about $1 \times 10^4$ to about $1 \times 10^8$ International Units (IU) or more.

Any of the known bulking agents heretofore employed in the lyophilization of biologically active materials can be employed herein, the polyhydric sugar alcohols being preferred. Examples of such sugar alcohols are those of the trihydric or higher alcohols such as glycerin, erythritol, arabitol, xylitol, sorbitol and mannitol, the last-mentioned being especially preferred. These and other bulking agents can be used singly or in combination. The bulking agent is generally effective when used at a level of at least about 15 wt.%, and preferably at a level of from about 25 to about 60 wt.%, of the interferon.

The stabilizing component of the lyophilized interferon composition can be selected from among any of the substances known in the art to improve the stability of the interferons. Especially suitable in this role are the amino acids glycine and alanine. Human serum albumin (HSA) also functions well as a preservative, or stabilizer, for the interferons. Since HSA is also effective as a bulking agent, it may be used to satisfy both the requirement for a bulking agent and the requirement for a stabilizer in the lyophilized formulations of this invention at one and the same time although, of course, where present, some other bulking agent, e.g., mannitol, can also be used. Combinations of the foregoing stabilizers, e.g., glycine and HSA are also within the scope of this invention. The effective amounts of stabilizer to be employed in a given formulation can vary widely and, of course, will be chosen to provide at least an interferon-stabilizer amount. When HSA is chosen as both the stabilizer and bulking agent, it will usually be necessary to employ more of this material than would be the case where it functions solely as a stabilizer and some other material, e.g., mannitol, is employed as the principal bulking agent. As a general guide. the stabilizer can be present at a level of from about 1 to about 150 milligrams, and preferably from about 10 to about 25 milligrams, per milliliter of reconstituted injectable interferon preparation.

The requirement for a dispersant can be satisfied with essentially any of the pharmaceutically acceptable surface active agents known to be effective dispersants for lyophilized interferons. These surface active agents are generally anionic or nonionic in nature. A preferred class of surface active agents are, e.g., the water-soluble esters derived from the reaction of a fatty acid such as lauric, palmitic, stearic, etc., acids with ethoxylated alcohols. These products, which are nonionic in character, are available from numerous commercial sources. Polysorbate 20, a polyoxyethylene sorbitan monolaurate prepared with about 20 moles of ethylene oxide per mole of sorbitol, is a specific representative of the preferred class of dispersants which have been found to provide generally good results. As with the other components of the lyophilized interferon composition of this invention, the amount of surface active agent dispersant can vary widely. Amounts ranging from about 0.001 w/v% to about 0.5 w/v% are usually effective to achieve long term dispersion of the lyophilized interferon.

The isotonic agent employed herein is preferably selected from among the pharmaceutically acceptable inorganic salts such as sodium chloride, potassium chloride, lithium chloride and magnesium chloride. Of these, sodium chloride and potassium chloride are especially preferred. The amount of salt present can vary widely, amounts ranging from about $1 \times 10^{-11}$ to about $3 \times 10^{-10}$ mole per unit of interferon being generally effective.

A buffer system will be present in an amount calculated to maintain a pH favorable to the long term storage stability of the lyophilized interferon composition, for example, a pH of from about 4.0 to about 8.0 and preferably from about 7.0 to about 7.4. The buffer system can be based on inorganic acids and their salts such as a combination of sodium dibasic phosphate and sodium monobasic phosphate but is preferably based on organic acids and the salts thereof such as citrate buffers (e.g. monosodium citrate-disodium citrate mixture, citric acid-trisodium citrate mixture, citric acid-monosodium citrate mixture, etc.), succinate buffers (e.g. succinic acid-monosodium succinate mixture, succinic acid-sodium hydroxide mixture, succinic acid-disodium succinate mixture, etc.), tartrate buffers (e.g. tartaric acid-sodium tartrate mixture, tartaric acid-potassium tartrate mixture, tartaric acid-sodium hydroxide mixture, etc.), fumarate buffers (e.g. fumaric acid-monosodium fumarate mixture, fumaric acid-disodium fumarate mixture, monosodium fumarate-disodium fumarate mixture, etc.), gluconate buffers (e.g. gluconic acid-sodium gluconate mixture, gluconic acid-sodium hydroxide mixture, gluconic acid-potassium gluconate mixture, etc.), oxalate buffers (e.g. oxalic acid-sodium oxalate mixture, oxalic acid-sodium hydroxide mixture, oxalic acid-potassium oxalate mixture, etc.), lactate buffers (e.g. lactic acid-sodium lactate mixture, lactic acid-sodium hydroxide mixture, lactic acid-potassium lactate mixture, etc.) and acetate buffers (e.g. acetic acid-sodium acetate mixture, acetic acid-sodium hydroxide mixture, etc.).

In addition to a bulking agent, stabilizer (or combined bulking agent/preservative as the case may be), dispersant, isotonic agent and buffer, each of which is critical to achieving the excellent storage stability properties of the lyophilized interferon composition of this invention, there may optionally be present the customary amounts of one or more other ingredients, as for example, pharmaceutically acceptable mild reducing agents, chelating agents, acid sugars, saccharides, etc., as is known from European Patent Application 0 123 291, discussed above, and other publications.

In general, any of the pre-lypholization and lyophilization procedures known in the art, for example, those described in U.S. Patent No. 4,496,537, can be followed herein with appropriate modifications readily apparent to those skilled in the art to provide the lyophilized interferon composition of this invention. As applied to the lyophilized interferon composition herein, these procedures are as follows:

1. Charge a portion of Water for Injection, USP, to a suitable vessel equipped with an agitator.

2. Charge and dissolve with agitation the components of the buffer system.

3. Charge and dissolve with agitation the bulking agent.

4. Charge and dissolve with agitation the preservative and dispersing agent.

5. Charge and dissolve the isotonic agent.

6. Charge and dissolve the Alpha-2 Interferon with gentle agitation.

7. Bring the batch to final volume with Water for Injection, USP.

8. In a sterile area, aseptically filter the solution into a sterilized vessel through a sterilized 0.2 micron filter which has been washed and tested for integrity. Test the integrity of the filter after filtration.

9. Aseptically fill the solution into sterilized vials and half-stoppered.

10. Aseptically load the filled vial into a sterilized shelf type lyophilizer.

11. Aseptically lyophilize the frozen solution.

12. Aseptically stopper the vials.

The sequence of these steps is not critical and can be altered in a variety of ways. The lyophilization operation (using a shelf lyophilizer) can usually be carried out as follows:

1. Precool the shelves to 5°C.

2. Load the vials in the chamber and cool to freeze.

3. Allow the solutions to freeze. Cool the condenser to -45°C or below. Start the vacuum.

4. Increase shelf temperatures at a relatively constant rate.

5. When the product temperature is at or above +25°C. for 2 hours, flood the chamber with sterile nitrogen until it reaches atmospheric pressure. Stopper the vials in the chambers.

6. Remove the vials from the chamber and seal them.

Employing the foregoing lyophilization procedure, interferon compositions were prepared with the formulations described in the following examples.


EXAMPLE 1


To prepare a 1 liter batch of alpha-2 interferon formulation for lyophilization, introduce approximately 8.8g of sodium chloride into an appropriate container. Taking into account that the active ingredient will be introduced into the formulation in a 0.05 molar succinate buffer vehicle, weigh into the container enough sodium succinate hexahydrate and succinic acid in an approximate ratio of 3.5 to 1 to give a final succinate buffer concentration of approximately 0.05 molar when diluted up to 1 liter.

Add sufficient purified water to the container to dissolve the sodium chloride, sodium succinate hexahydrate, and succinic acid being sure to leave sufficient room for the remaining ingredients to be added. Add in the form of a concentrate, sufficient polysorbate 20 in sterile water to give a final concentration in the formulation of approximately 0.3 grams per liter. Add in the form of a concentrate, sufficient human serum albumin (HSA) to give a final concentration in the formulation of approximately 2 grams per liter. Add in the form of a concentrate sufficient recombinant alpha-2 interferon in a 0.05 molar succinate buffer (approximately 3.5 parts sodium succinate hexahydrate to approximately 1 part succinic acid) to provide a final interferon concentration of approximately $6 \times 10^7$ units per milliliter of formulation. Add sufficient sterile water to make a final formulation volume of 1 liter. Mix the solution gently to uniformity and adjust pH if necessary to approximately 4.9 with hydrochloride acid or sodium hydroxide.

Aseptically filter the solution, fill into sterilized vials and half-stopper. Place the vials in the chamber of a precooled (approximately 5°C) shelf lyophilizer and allow the contents of the vials to freeze. When the condensor is at -45°C or below, start the vacuum while increasing the temperature at a relatively constant rate. When the contents of the vials have reached +25°C for 2 hours, introduce sterile nitrogen to the chamber until ambient pressure has been achieved. Stopper the vials, remove them from the chamber and seal the vials.

EXAMPLES 2-4

Employing substantially the same preparative techniques described in Example 1, lyophilized recombinant alpha-2 interferon preparations are prepared from the following formulations:

| Ingredient | Concentration/ml |
|---|---|
| Recombinant Alpha-2 Interferon | |
| Human Serum Albumin | 2.00 mg/ml |
| Sodium Chloride | 8.77 mg/ml |
| Polysorbate 20 | 0.30 mg/ml |
| Sodium Succinate Hexahydrate | 8.19 mg/ml |
| Succinic Acid | 2.33 mg/ml |
| Disodium Ethylenediamine- | |
| tetraacetic Acid Dihydrate | |
| (Chelating Agent) | 0.28 mg/ml |
| Sterile Water for | |
| Injection, USP qsad | |
| pH adjustment | 4.9 ± 0.1 |

| Ingredient | Concentration/ml |
|---|---|
| Recombinant Alpha-2 Interferon | |
| Mannitol | 40.0 mg/ml |
| Glycine | 2.25 mg/ml |
| Sodium Chloride | 1.75 mg/ml |
| Polysorbate 20 | 0.30 mg/ml |
| Sodium Succinate Hexahydrate | 8.19 mg/ml |
| Succinic Acid | 2.33 mg/ml |
| Sterile Water for | |
| Injection, USP qsad | |
| pH adjustment | 4.9 ± 0.1 |

| Ingredient | Concentration/ml |
|---|---|
| Recombinant Alpha-2 Interferon | |
| Mannitol | 40.0 mg/ml |
| Glycine | 2.25 mg/ml |
| Sodium Chloride | 1.75 mg/ml |
| Polysorbate 20 | 0.30 mg/ml |
| Sodium Succinate Hexahydrate | 8.19 mg/ml |
| Succinic Acid | 2.33 mg/ml |
| Disodium Ethylenediamine-tetraacetic Acid Dihydrate | 0.28 mg/ml |
| Sterile Water for Injection, USP qsad | |
| pH adjustment | 4.9 ± 0.1 |

Other interferons and lymphokines generally can be provided in lyophilized form employing much the same procedures and with much the same results.

Since the lyophilized interferon preparations of this invention do not require refrigeration to substantially retain their initial activities for up to two years or more, they are advantageously provided as part of a kit containing all that is required for the parenteral administration of a single or multiple dosage. Thus, for example, the lyophilized interferon and the preserved sterile water required for its reconstitution can be stored, reconstituted when needed and administered employing the two-component syringes described in U.S. Patent Nos. 4,599,082 or 4,613,326, the disclosures of which are incorporated by reference herein.

## Claims

1. A lyophilized lymphokine composition for therapeutic administration which comprises:
   a) a therapeutically effective amount of a lymphokine;
   b) a bulking agent;
   c) a stabilizer for the lymphokine;
   d) a dispersant;
   e) an isotonic agent; and
   f) a buffer to maintaining the pH of the composition within a range which is conductive to the stability of the lymphokine component thereof.

2. The lyophilized composition of claim 1 where the lymphokine is an interferon.

3. The lyophilized composition of claim 1 wherein the bulking agent is a polyhydric sugar alcohol.

4. The lyophilized composition of claim 1 wherein the stabilizer is selected from the group consisting of glycine and alanine.

5. The lyophilized composition of claim 1 wherein the dispersant is a nonionic surface active agent.

6. The lyophilized composition of claim 1 wherein the buffer is a combination of an organic acid and an acid salt of an organic acid.

7. A lyophilized composition for therapeutic administration which comprises:
   a) a therapeutically effective amount of an interferon;
   b) a bulking agent selected from the group consisting of polyhydric sugar alcohol and human serum albumin;
   c) a stabilizer for the interferon selected from the group consisting of glycine, alanine and human serum albumin;
   d) a nonionic surface active agent as dispersant;
   e) an inorganic salt as isotonic agent; and,

f) a buffer based on an organic acid and an acid organic acid salt to maintain the pH of the composition within a range of from about 4.0 to about 8.0.

8. The lyophilized composition of claim 7 wherein the interferon is an alpha interferon.

9. The lyophilized composition of claim 2 or 7 wherein the interferon is a recombinant alpha interferon.

10. The lyophilized composition of claim 1 or 7 wherein the polyhydric sugar alcohol bulking agent is selected from the group consisting of glycerine, erythritol, arabitol, xylitol, sorbitol and mannitol.

11. The lyophilized composition of claim 1 or 7 wherein both the bulking agent and the stabilizer is human serum albumin.

12. The lyophilized composition of claim 1 or 7 wherein the nonionic surface active agent dispersant is a polyoxyethylene sorbitan monolaurate.

13. The lyophilized composition of claim 1 or 7 wherein the inorganic salt isotonic agent is selected from the group consisting of sodium chloride and potassium chloride.

14. The lyophilized composition of claim 1 or 7 wherein the buffer is a combination of succinic acid and an alkali metal succinate.

15. The lyophilized composition of claim 1 or 7 further comprising at least one additional pharmaceutically acceptable component selected from the group consisting of mild reducing agent, chelating agent, acid sugar and saccharide.

16. The lyophilized composition of claim 1 or 7 suitable for parenteral administration upon reconstitution.

17. A two-component syringe wherein one component is the lyophilized composition of claim 1 and the other component is an amount of water sufficient for the reconstitution of the lyophilized composition.

18. A two-component kit wherein one component is the lyophilized composition of claim 1 and the other component is a vial containing preserved sterile water for reconstitution of the lyophilized composition.

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 089 245 (INTER-YEDA LTD) <br> * Claims 1-4; page 4, lignes 7-14 * | 1-18 | A 61 K 9/14 <br> A 61 K 47/00 <br> A 61 K 45/02 <br> A 61 K 37/02 |
| Y | EP-A-0 196 203 (SHERING CORP.) <br> * Claims 1,2,4-6 * | 1-18 | |
| Y | WO-A-8 504 328 (CETUS CORP.) <br> * Page 11 * | 1-18 | |
| Y,D | EP-A-0 123 291 (KYOWA HAKKO KOGYO CO. LTD) <br> * Claims 1-5; page 4, lines 20-21 * | 1-18 | |
| X,P | EP-A-0 229 016 (SHIONOGI KABUSHIKI K.K.) <br> * Claims * | 1,3,5, 10,11, 12,16 | |
| A | CHEMICAL ABSTRACTS, vol. 90, no. 13, 26th March 1979, page 416, abstract no. 101740e, Columbus, Ohio, US; J.J. SEDMAK et al.: "Thermal and vortical stability of purified human fibroblast interferon", & ADV. EXP. MED. BIOL. 1978, 110(HUM. INTERFERON: PROD. CLIN. USE), 133-52 <br> * Abstract * | 1-18 | TECHNICAL FIELDS SEARCHED (Int. Cl.4) <br><br> A 61 K 9/00 <br> A 61 K 47/00 <br> A 61 K 45/00 <br> A 61 K 37/00 |
| A | US-A-4 469 228 (SCHERING CORP.) <br> * Claims * | 1-18 | |

The present search report has been drawn up for all claims

| Place of search | Date of completion of the search | Examiner |
|---|---|---|
| THE HAGUE | 22-07-1988 | GERLI P.F.M. |